# EUROPÄISCHE PATENTSCHRIFT

(11) **EP 1 174 097 B1**
(45) Veröffentlichungstag und Bekanntmachung des Hinweises auf die Patenterteilung: **05.07.2006**
(21) Anmeldenummer: 01114920.0
(22) Anmeldetag: 20.06.2001
(51) Int. Cl.: A61F 2/01, A61B 17/00

(54) **Vorrichtung zum Verschliessen einer Verbindung zwischen der Aorta und der Arteria pulmonalis**
Device for occluding the connection between aorta and arteria pulmonalis
Dispositif pour fermer la communication entre l'aorte et le conduit pulmonalis

(30) Priorität: 20.07.2000 DE 10035230
(43) Veröffentlichungstag der Anmeldung: 23.01.2002
(73) Patentinhaber: Dr. Osypka GmbH, 79618 Rheinfelden (DE)
(72) Erfinder: Osypka, Peter, Dr., 79618 Rheinfelden (DE)
(74) Vertreter: Maucher, Wolfgang

(56) Entgegenhaltungen:
- EP-A- 0 556 564
- EP-A- 0 698 373
- WO-A-95/28885
- DD-A- 233 303
- DE-A- 3 116 462
- DE-A- 19 607 451
- US-A- 5 192 301

## Beschreibung

Die Erfindung betrifft eine Vorrichtung zum Verschließen einer nach der Geburt offen gebliebenen Verbindung zwischen der Aorta und der Arteria pulmonalis (Lungenarterie) mit einem in diese Verbindung mittels Katheter einführbaren Verschlußelement, wobei das Verschlußelement zwei beabstandete, über ein Zugelement elastisch verbundene Verschlüsse aufweist, die in Gebrauchsstellung die beiden Mündungen der zwischen der Aorta und der Arteria pulmonalis befindlichen Verbindung abdecken oder ausfüllen.

Es ist bekannt, daß sich eine zwischen der Aorta und der Lungenarterie zunächst offene Verbindung nach der Geburt normalerweise von selbst schließt. In manchen Fällen bleibt diese Verbindung jedoch offen und muß dann künstlich verschlossen werden, was bisher auf chirurgischem Wege erfolgt.

Aus US-A 5 192 301 ist eine Vorrichtung der eingangs genannten Art bekannt. Das Verschlußelement kann dabei mit einem Schubdraht in seine Postition gebracht werden. Dabei besteht die Gefahr, daß die bestmögliche Platzierung des Verschlußelements nicht getroffen wird.

Die Vorrichtung gemäß des Oberbegriffs von Anspruch 1 ist aus der Patent schrift DD233303 bekannt.

Bei der Verwendung dieses Systems an der Arteria pulmonalis können sich eventuell in diese hineinragende Teile ergeben, an denen es zu unerwünschten Blutschädigungen und eventuell zu einer Trombenbildung kommen könnte.

Es besteht deshalb die Aufgabe, eine Vorrichtung der eingangs genannten Art zu schaffen, mit welcher eine nach der Geburt offen bleibende Verbindung zwischen der Aorta und der Arteria pulmonalis dicht verschlossen werden kann, wobei nach dem Einführen des Verschlußelements die bestmögliche Platzierung gesucht und erreicht werden kann.

Die Lösung dieser Aufgabe besteht darin, daß die Vorrichtung im wesentlichen durch ein mittels Katheter einführbares Verschlußelement mit zwei beabstandeten Verschlüssen der eingangs genannten Art gebildet ist, welches dadurch gekennzeichnet ist, daß ein Verschluß und das Zugelement eine durchgehende, bis in die Nähe des anderen Verschlusses reichende Innenlängshöhlung für den Eintritt eines Mandrins oder dergleichen Schiebe- und/oder Drehwerkzeuges hat, die an ihrem Ende ausgesteift und/oder armiert ist, und daß im Bereich des Eintrittes in die Innenlängshöhlung des Zugelements eine Armierung angeordnet ist, an welcher eine über den unmittelbar benachbarten Verschluß nach außen überstehende Öse und ein an dieser eingehängter Zugfaden angeordnet sind.

Auf diese Weise kann ohne chirurgische Öffnung des Brustkorbes mit Hilfe eines Katheters ein entsprechendes Verschlußelement in die Öffnung gebracht werden, wo diese unabhängig von ihrer Länge beidseits verschlossen wird. Das zwischen den beiden Verschlüssen angeordnete elastische Zugelement erlaubt dabei eine Anpassung an Längenunterschiede der entsprechenden Öffnung. Gleichzeitig werden beide Verschlüsse durch dieses elastische Zugelement an ihren "Sitz" gedrückt und ergeben eine gute Abdichtung. Es kann also eine gute Anpassung sowohl an die Länge als aber auch an den Durchmesser der Öffnung erreicht werden und ein Restfluß zwischen Aorta und Arteria pulmonalis (Lungenarterie) wird vermieden.

Die armierte Innenlängshöhlung erlaubt dabei die Benutzung eines Mandrins oder dergleichen Werkzeuges beim Einführen. Der an der Öse an dem proximalen Verschluß oder "Schirm" angeordnete Zugfaden kann als "Sicherheitsleine" angesehen werden. Erst wenn nach dem Einführen des Verschlußelementes geprüft ist, daß die Verschlüsse des Verschlußelementes richtig platziert sind, kann dieser Faden durch Ziehen an einem Ende entfernt werden. Andernfalls kann der gesamte Verschluß durch Ziehen an beiden Enden dieses durch die Öse geführten Fadens und des Katheters wieder entfernt werden. Auch kann durch abwechselndes Betätigen des Zugfadens und des Mandrins oder Werkzeuges die bestmögliche Platzierung gesucht und erreicht werden. Das Verschlußelement kann also von einer Seite her in die zu schließende Öffnung eingeführt und so platziert werden, daß der eine Verschluß das in Einführrichtung entferntere Ende bzw. die Mündung dieser Öffnung erreicht und sie verschließt. Der zweite Verschluß überdeckt dann die Eintrittsöffnung und das Einführwerkzeug kann wieder zurückgezogen werden.

Zweckmäßig ist es dabei, wenn die Armierung im Bereich des Eintrittes in die Innenlängshöhlung des Zugselementes eine Wendel vorzugsweise aus Metalldraht ist. Somit bietet sie der Öse vor allem bei dem Aufbringen von Zugkräften einen guten Halt.

Zweckmäßig ist es dabei, wenn die Verschlüsse und das oder die sie verbindenden Zugelemente aus elastischem Kunststoff, insbesondere aus Silikon-Material, bestehen. Silikon hat sich seit langen Jahren beispielsweise auch bei implantierbaren Herzschrittmacherelektroden als allergiefrei und körperverträglich bewährt. Gleichzeitig ist es gummielastisch und kann deshalb die Verschlüsse selbst und das oder die Zugelemente selbsttätig an anatomische Gegebenheiten anpassen.

Dabei können die beiden Verschlüsse und das sie elastisch verbindende Zugelement einstückig verbunden sein. Entsprechend einfach ist auch die Einführung und plazierung dieses Verschlußelementes.

Die beiden Verschlüsse des Verschlußelementes können platten- oder schirmartig ausgebildet und etwa eben oder wenigstens bereichsweise an ihrer äußeren Oberfläche konkav eingesenkt sein. Dadurch wird gegenüber nach außen gewölbten Verschlüssen eine Verengung des Querschnittes der Aorta und der Arteria pulmonalis vermieden.

Für eine sichere Einführung des Verschlußelementes ist es zweckmäßig, wenn die Innenlängshöhlung des Zugelementes, insbesondere durch eine Wendel zum Beispiel aus Metalldraht, innenseitig armiert ist und daß die Armierung an ihrem Ende eine innenseitige hohle Profilierung bildet oder umgrenzt, beispielsweise einen Schlitz, in den das flache Ende des Mandrins oder Werkzeuges paßt. Somit kann der Benutzer das Verschlußelement mit Hilfe des Mandrins oder Werkzeuges vorschieben und dabei gegebenenfalls auch etwas verdrehen, bis die Verschlüsse ihre vorgesehene Lage an den beiden voneinander entfernt liegenden Mündungen der Öffnung erreicht haben und diese sicher verschließen.

Die schirmartigen Verschlüsse können gegen die Elastizität ihres Materiales oder Werkstoffes faltbar und in einen schlauchartigen Zuführkatheter - der zu der Vorrichtung gehören kann - einsetzbar sein, dessen Innenquerschnitt der Außenabmessung der zusammengefalteten Verschlüsse entspricht. Somit kann das gesamte Verschlußelement mit beiden Verschlüssen zunächst in gefalteter Anordnung innerhalb des Katheters in den Bereich der Öffnung gebracht und dort dann mit Hilfe eines ebenfalls durch den Katheter vorschiebbaren Mandrins oder Werkzeuges aus dem Zuführkatheter heraus in die Öffnung eingeschoben werden.

Die schirmartigen Verschlüsse können eine runde oder etwa viereckige oder ovale Umfangskontur haben und ihre Fläche kann insbesondere Aussteifungsrippen und/oder -stege aufweisen. Somit sind Anpassungen an unterschiedliche anatomische Gegebenheiten im Bereich der zu verschießenden Öffnung möglich.

Eine weitere Ausgestaltung der Erfindung zur Vergrößerung der Sicherheit der Abdichtung der zu verschließenden Öffnung kann darin bestehen, daß das Zugelement zwischen den beiden endseitigen Verschlüssen wenigstens einen weiteren elastischen Verschluß trägt, der zumindest in seinem Randbereich elastisch anpaßbar ist. Dieser weitere Verschluß kann dann also zwischen den beiden endseitigen Verschlüssen im Verlauf der zu verschließenden, etwa kanalförmigen Öffnung an deren Innenwand anliegen und einen ungewollten Blutfluß durch diese Öffnung also noch weitergehend unterbinden.

Dabei können an dem Zugelement zwischen den beiden endseitigen Verschlüssen zwei zueinander beabstandete weitere Verschlüsse angeordnet sein, deren Außendurchmesser insbesondere kleiner als der der endseitigen Verschlüsse ist, damit sie in das Innere der kanalförmigen Öffnung passen.

Der oder die zusätzlichen Verschlüsse im Verlauf des Zugelementes können ebenfalls etwa schirmartig und gegen eine Rückstellkraft des Werkstoffes faltbare, schirmartige oder konische Form haben. Somit können sie ebenfalls innerhalb des Zuführkatheters untergebracht werden.

Welche Verschlußform und -größe gewählt wird, kann gegebenenfalls vor dem Eingriff zum Beispiel durch Ultraschall festgestellt werden. Auch lassen sich die schirmartigen Verschlüsse nach der Feststellung der Größe der zu verschließenden Öffnung beziehungsweise des "Ductus" zurechtschneiden, was vor allem bei einer Fertigung aus Silikon mit einer einfachen Schere am Operationstisch durchgeführt werden könnte.

Insgesamt ergibt sich ein einfach einführbarer Verschluß, dessen beiden Verschlüsse selbst bei großen anatomischen Anomalien für eine sichere und dichte Fixierung sorgen.

Nachstehend sind Ausführungsbeispiele anhand der Zeichnung näher beschrieben. Es zeigt in zum Teil schematisierter Darstellung:
- Fig. 1: eine Person und deren Herz, wobei angedeutet ist, daß eine nach der Geburt offen gebliebene Verbindung zwischen der Aorta und der Lungenarterie mit einem mittels Katheter einführbaren Verschlußelement nachträglich verschlossen ist,
- Fig. 2: in vergrößertem Maßstab einen Schnitt durch das Herz sowie durch Teile der Aorta und der Lungenarterie oder Arteria pulmonalis mit einer offen gebliebenen, aber nachträglich mit einem erfindungsgemäßen Verschlußelement verschlossenen Verbindung dieser beiden Blutgefäße,
- Fig. 3: in vergrößertem Maßstab das Verschlußelement in Gebrauchslage, wobei das Verschlußelement zwei beabstandete und mittels Zugelement elastisch verbundene Verschlüsse für die Mündungen oder Enden der Verbindung zwischen der Aorta und der Arteria pulmonalis aufweist,
- Fig. 4: in vergrößertem Maßstab die in Fig. 3 mit dem Kreis A markierte Einzelheit, sowie
- Fig. 5 bis 18: jeweils Draufsichten und Seitenansichten von zu dem Verschlußelement gehörenden Verschlüssen unterschiedlicher Außenkontur.

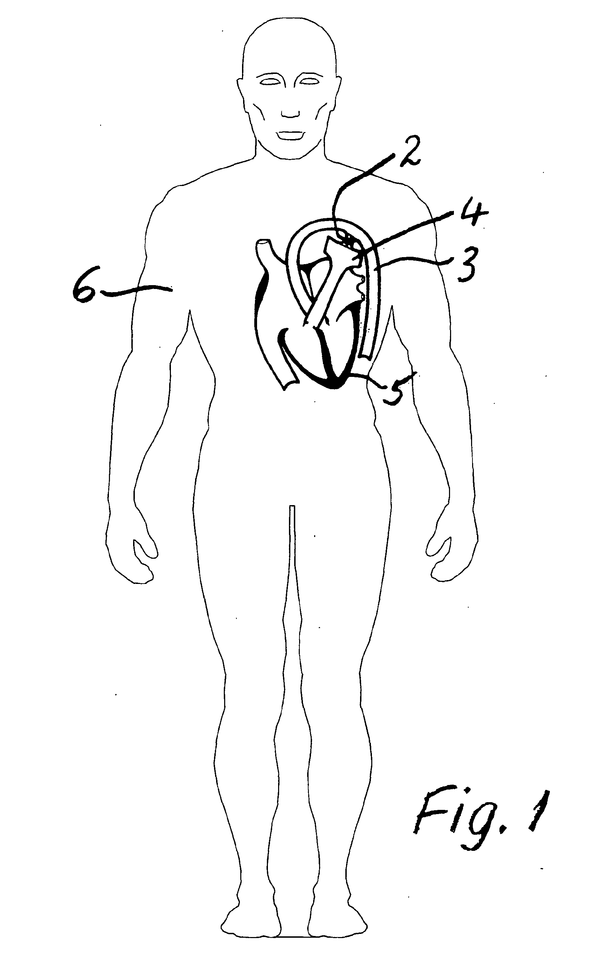

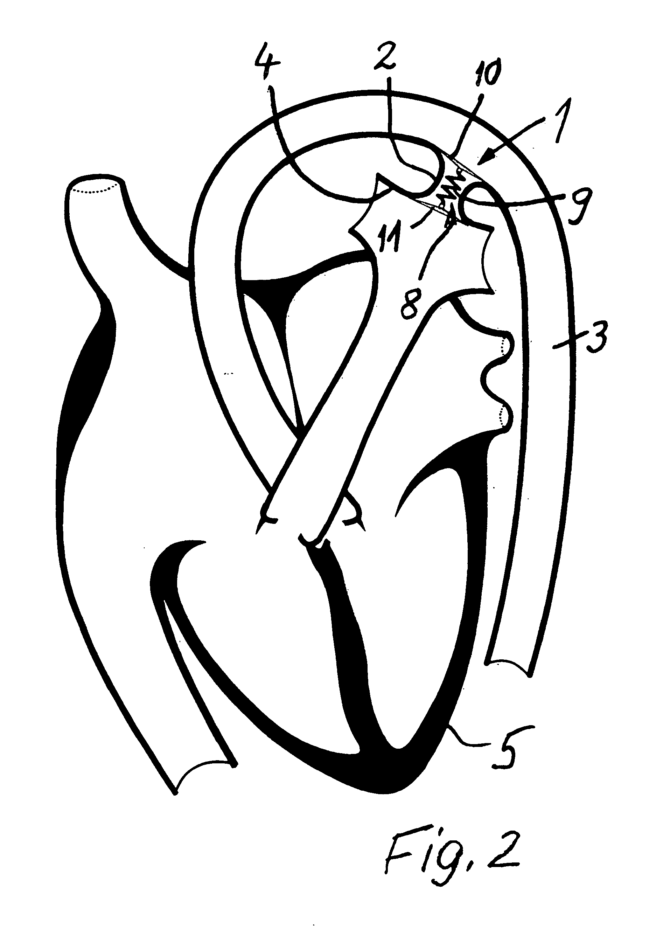

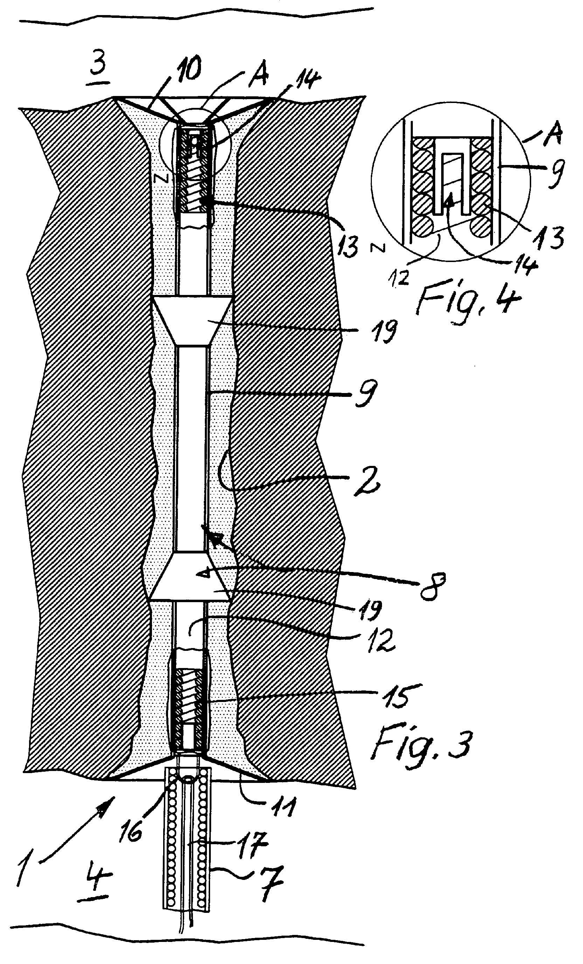

Eine im ganzen mit 1 bezeichnete, besonders gut in Fig. 3 erkennbare Vorrichtung dient zum Verschließen einer nach der Geburt offen gebliebenen Verbindung 2 zwischen der Aorta 3 und der Lungenarterie oder Arteria pulmonalis 4, wobei man diese Verbindung 2 in Fig. 1 und 2 am Herzen 5 einer Person 6 schematisiert dargestellt erkennen kann. Dabei wird die Vorrichtung 1 im wesentlichen gebildet durch ein mittels Katheter 7 einführbares Verschlußelement 8, so daß ein chirurgischer Eingriff zum Verschließen der Verbindung 2 vermieden werden kann. Von dem erwähnten Katheter 7 ist dabei nur dessen Ende in Fig. 3 angedeutet, jedoch ist dieses Ende so gestaltet, daß es das gesamte Verschlußelement 8 in sich aufnehmen kann, um durch Blutgefäße an die Einsatzstelle gebracht werden zu können.

In Fig. 2 und vor allem in Fig. 3 ist dargestellt, daß das Verschlußelement 8 zwei beabstandete, über ein elastisches Zugelement 9 verbundene, aber beabstandete Verschlüsse 10 und 11 aufweist, die in Gebrauchsstellung die beiden Mündungen der zwischen der Aorta 3 und der Arteria pulmonalis 4 befindlichen Verbindung 2 abdecken oder ausfüllen. Fig. 3 deutet dabei an, daß diese Mündungen der Verbindung 2 gegebenenfalls gegenüber dem übrigen Verlauf dieser kanalartigen Verbindung 2 etwas erweitert sind, trotzdem aber von den Verschlüssen 10 und 11 abgedeckt werden, so daß ein Flüssigkeitsdurchtritt unterbunden wird.

Während Fig. 2 schematisiert andeutet, daß die beiden Verschlüsse 10 und 11 durch eine Feder als Zugelement 9 verbunden sein könnten, ist gemäß Fig. 3 vorgesehen, daß die Verschlüsse 10 und 11 und das sie verbindende Zugelement 9 aus elastischem Kunststoff, bevorzugt aus Silikon-Material bestehen, welches sich beim Einsatz innerhalb des menschlichen Körpers als inert und allergiefrei bewährt hat.

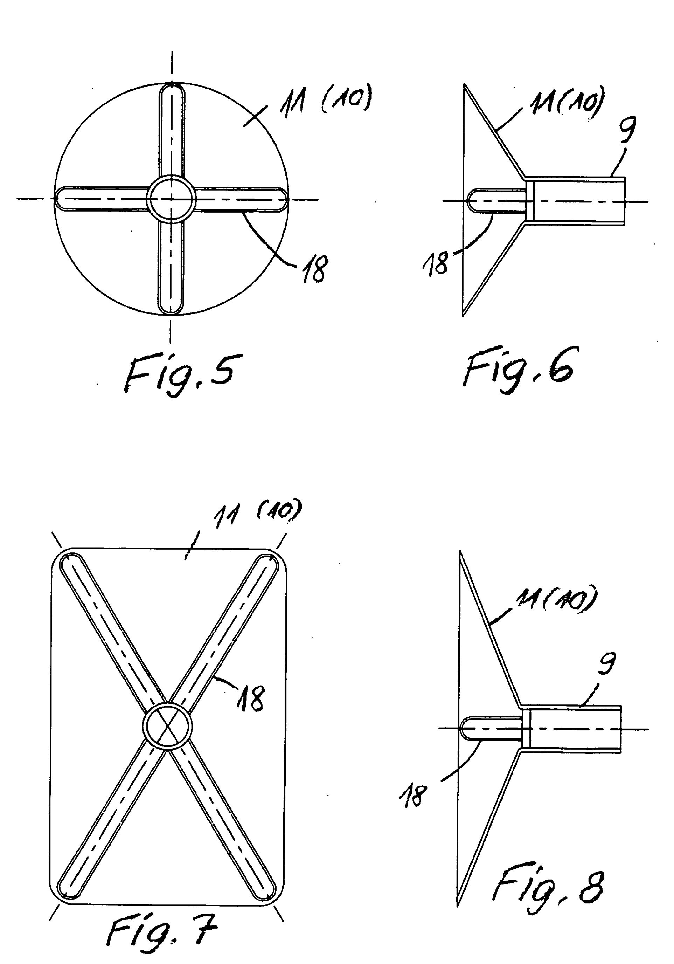

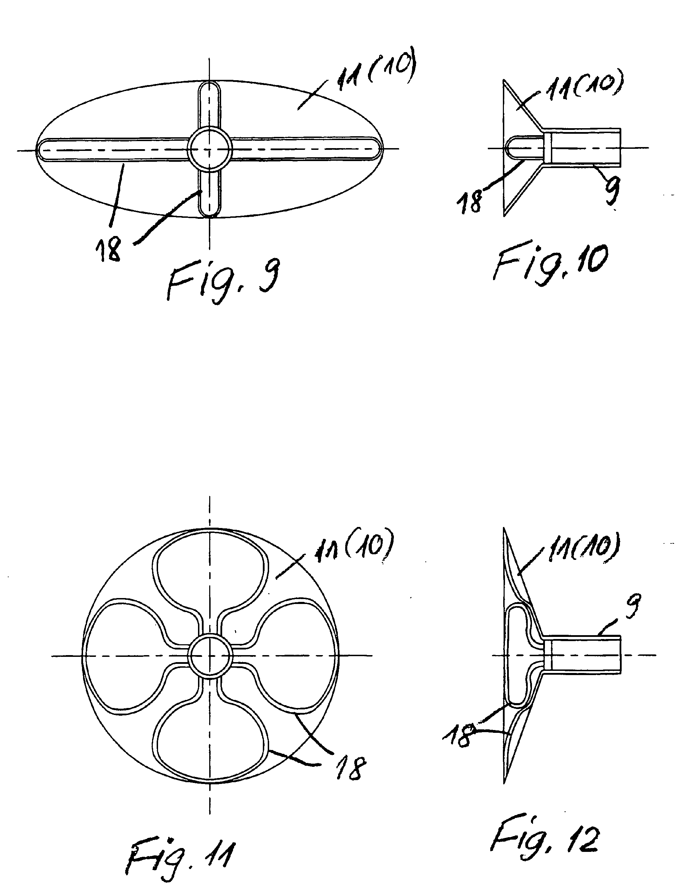

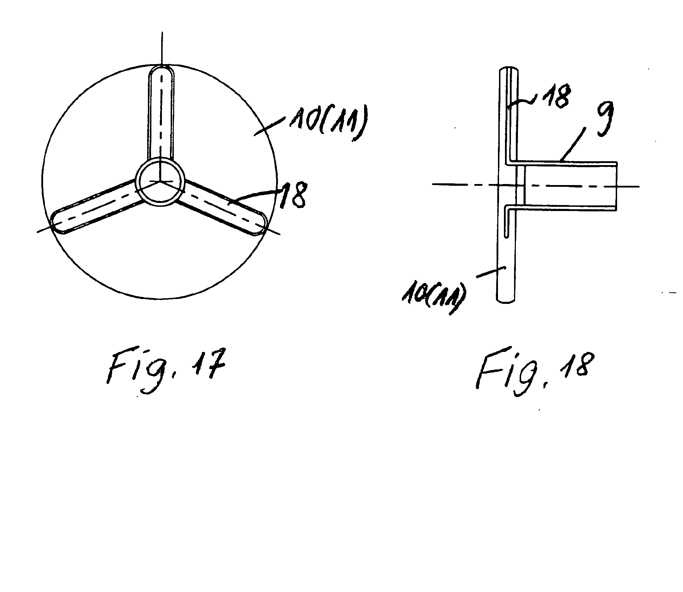

Dabei sind die beiden Verschlüsse 10 und 11 und das sie elastisch verbindende Zugelement 9 im Ausführungsbeispiel einstückig verbunden. Die beiden Verschlüsse 10 und 11, die jeweils etwa die gleiche Form und den gleichen Aufbau haben können und von denen unterschiedliche Beispiele hinsichtlich Form und Aufbau in den Figuren 5 bis 18 dargestellt sind, sind dabei jeweils etwa schirmartig ausgebildet, das heißt sie können gegenüber den dargestellten Formen und auch gegenüber der Anordnung in Fig. 3 auf einen erheblich geringeren Querschnitt zusammengefaltet werden, damit sie in den Katheter 7 hineinpassen. In Gebrauchsstellung sind sie eben oder, wie in den Ausführungsbeispielen dargestellt, an ihrer äußeren Oberfläche konkav eingesenkt, so daß an beiden Blutgefäßen 3 und 4 kein Vorsprung durch diese Verschlüsse 10 und 11 entsteht.

In Fig. 3 erkennt man, daß der eine Verschluß 11, nämlich der proximale Verschluß 11 und das Zugelement 9 eine durchgehende, bis in die Nähe des anderen Verschlusses 10 reichende Innenlängshöhlung 12 für den Eintritt eines Mandrins oder dergleichen Schiebe- und/oder Drehwerkzeuges hat, die dabei zumindest am Ende im Bereich der Einzelheit A (vgl. auch Fig. 4) ausgesteift und armiert ist. Somit kann durch den Katheter 7 ein entsprechendes, in der Zeichnung nicht näher dargestelltes Schiebewerkzeug durch das Verschlußelement 8 hindurch bis nahe zu dem Verschluß 10 eingeführt werden, um das Verschlußelement aus dem Katheter 7 in die Verbindung 2 hineinzuschieben. Der Verschluß 10 unterscheidet sich also von dem Verschluß 11 dadurch, daß er keine solche Eintrittsöffnung für ein solches Werkzeug hat.

Die Innenlängshöhlung 12 des Zugelementes 9 ist dabei durch eine Wendel 13, zum Beispiel aus einem Metalldraht innenseitig armiert und diese Armierung bildet an ihrem Ende nahe dem Verschluß 10 eine innenseitige hohle Profilierung, die gemäß Fig. 4 zusätzlich einen Schlitz 14 enthält, in den ein entsprechend flaches Ende des Mandrins oder Werkzeuges paßt, so daß auch Drehbewegungen zur Anpassung des Verschlusses 10 oder des gesamten Verschlußelementes 8 an bestimmte anatomische Gegebenheiten möglich sind.

Die schirmartigen Verschlüsse 10 und 11 sind gegen die Elastizität ihres Materiales oder Werkstoffes in nicht näher dargestellter Weise, wie schon erwähnt, faltbar und in den schlauchartigen Zuführkatheter 7 einsetzbar, bevor das Verschlußelement 8 an seine Einsatzstelle gebracht wird. Der Innenquerschnitt dieses Zuführkatheters 7 entspricht also den Außenabmessungen der zusammengefalteten Verschlüsse 10 und 11.

In Fig. 3 erkennt man ferner im Bereich des Eintrittes in die Innenlängshöhlung 12 des Zugelementes 9, also nahe dem Verschluß 11, eine Armierung in Form einer zweiten Wendel 15 beispielsweise aus Metalldraht. Dadurch wird das Zugelement 9 insbesondere nahe beiden Verschlüssen 10 und 11 stabilisiert. Ferner ist es dadurch möglich, an dieser Wendel 15 eine über den benachbarten Verschluß 11 nach außen überstehende Öse 16 vorzusehen, an der ein Zugfaden 17 eingefädelt und angehängt ist. Mit Hilfe dieses Zugfadens 17 kann durch eine Zugkraft an seinen beiden Enden das gesamte Verschlußelement 8 wieder aus der Verbindung 2 herausgezogen werden, falls beispielsweise eine Überprüfung mit Ultraschall zeigt, daß die Plazierung nicht stimmt oder die Dichtigkeit nicht mit der notwendigen Sicherheit erreicht wurde oder die Verschlüsse 10 und 11 nicht genau genug passen.

Ist ein gut abdichtendes und passendes Verschlußelement 8 eingesetzt, kann der Zugfaden 17 an einem Ende aus der Öse 16 entfernt werden.

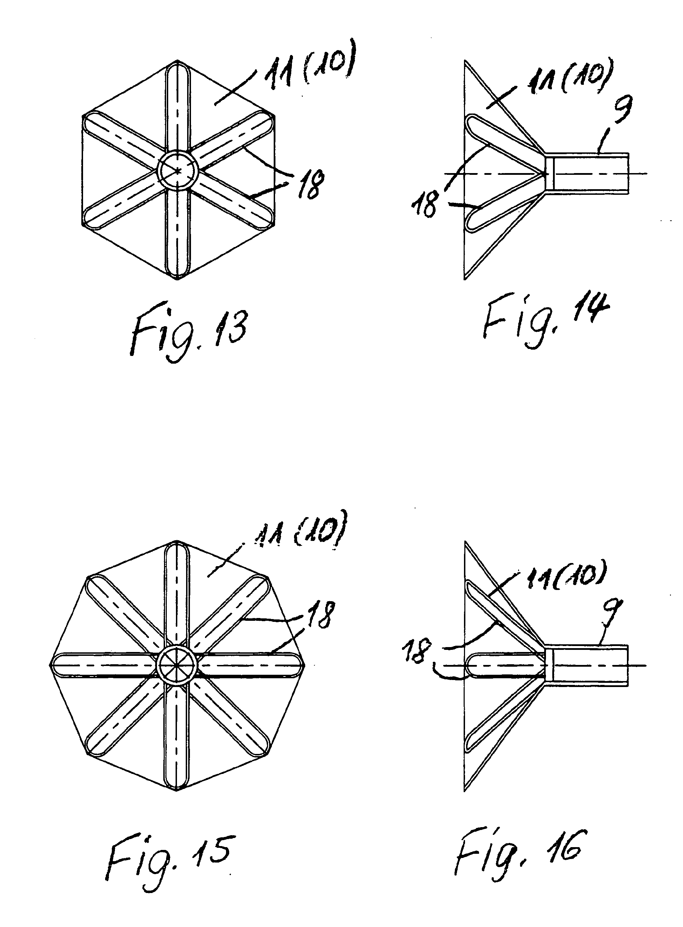

In den schon mehrfach erwähnten Figuren 5 bis 18 ist dargestellt, daß die schirmartigen, gegenüber einem Regenschirm allerdings entgegengesetzt konkav geformten (Fig. 5 bis 16) oder ebenen (Fig. 17 und 18) Verschlüsse 10 und 11 unterschiedlichste Umfangskonturen, beispielsweise runde, viereckige, vieleckige oder ovale Umfangskonturen haben können und daß ihre Fläche jeweils Aussteifungsrippen oder -stege 18 gegebenenfalls unterschiedlicher Anzahl aufweist. Man erkennt entweder vom Zentrum ausgehend drei (Fig. 17) oder vier (Fig. 5 bis 12) oder noch mehr (Fig. 13 bis 6) derartige Aussteifungsrippen oder -stege 18. Dabei können diese Rippen ihrerseits unterschiedlich geformt sein, wie es insbesondere in Fig. 11 im Vergleich zu den übrigen Figuren angedeutet ist. Somit kann die Steifigkeit und Anpaßbarkeit der Verschlüsse 10 und 11 je nach Anwendungsfall und anatomischen Verhältnissen unterschiedlich vorherbestimmt oder gewählt werden. Darüber hinaus können die Umfangskonturen auch zusätzlich vom Operateur beschnitten werden, insbesondere in den zwischen den Aussteifungsrippen oder -stegen 18 befindlichen Randbereichen.

In Fig. 3 erkennt man ferner, daß das Zugelement 9 zwischen den beiden endseitigen Verschlüssen 10 und 11 wenigstens einen, im Ausführungsbeispiel 2 zueinander beabstandete weitere insbesondere elastische Verschlüsse 19 trägt oder aufweist, die zumindest in ihren Randbereichen elastisch anpaßbar sind, sich also selbsttätig an den Innenquerschnitt der kanalartigen Verbindung 2 anpassen und anlegen können. Dabei sind deren Außendurchmesser im Ausführungsbeispiel kleiner als die der endseitigen Verschlüsse 10 und 11, um den in der Regel im Inneren der Verbindung 2 geringeren Querschnitt gegenüber den Enden oder Mündungen dieser Verbindung 2 zu berücksichtigen. Es ist vor allem durch Fig. 3 gut verdeutlicht, daß auf diese Weise ein Durchtritt von Flüssigkeit von dem einen Blutgefäß in das andere weitestgehend ausgeschlossen ist.

Die zusätzlichen Verschlüsse 19 sind gemäß Fig. 3 konisch und könnten dabei ebenfalls etwa schirmartig und gegen eine Rückstellkraft des Werkstoffes faltbar sein, um sich noch besser an unterschiedliche Innenkonturen innerhalb der Verbindung 2 anpassen zu können.

Nachdem per Ultraschall die Größe und Form der Verbindung 2 oder ihres Ductus festgestellt ist, kann also ein passendes Verschlußelement 8 gewählt und mit Hilfe des Katheters 7 eingeführt werden.

Gegebenenfalls kann dabei die Umfangskontur der Verschlüsse 10 und 11 vorher noch am Operationstisch zurechtgeschnitten werden. Während der Einführungsphase ist die Vorrichtung 1 zusammengefaltet in dem Schutz- oder Einführkatheter 7 untergebracht und wird an den gewünschten Ort geschoben und dort mit einem Mandrin oder Schubwerkzeug herausgedrückt. Das abgeflachte Ende dieses Werkzeuges paßt in den distalen Aufnahmeschlitz 14 am Ende der Innenlängshöhlung 12 des Zugelementes 9, so daß der distale Verschluß 10 bei Bedarf auch in die "richtige" Position gedreht werden kann. Vorteilhaft ist die schon erwähnte Öse 16 mit dem Faden 17, der praktisch eine "Sicherheitsleine" darstellt. Erst wenn nach dem Plazieren des Verschlußelementes 8 geprüft worden ist, ob es richtig sitzt, wird dieser Faden 17 durch Ziehen an einem seiner Enden entfernt. Anderenfalls kann das gesamte Verschlußelement 8 mit Hilfe dieses Fadens 17 und des Katheters 7 wieder entfernt werden. Die schirmartigen Verschlüsse 10 und 11 an beiden Enden des Verschlußelementes 8 sorgen selbst bei großen anatomischen Anomalien für eine sichere und dichte Fixierung.

Die Vorrichtung 1 dient zum Verschließen einer nach der Geburt einer Person 6 offen gebliebenen kanalartigen Verbindung 2 zwischen der Aorta 3 und der Arteria pulmonalis 4. Sie wird im wesentlichen durch ein mittels Katheter 7 einführbares Verschlußelement 8 gebildet, welches zwei beabstandete und über ein elastisches Zugelement 9 verbundene Verschlüsse 10 und 11 aufweist, die ihrerseits eine gewisse Elastizität haben und in Gebrauchsstellung die beiden Mündungen oder Enden der zwischen der Aorta 3 und der Arteria pulmonalis 4 befindlichen Verbindung 2 abdecken oder ausfüllen, wobei es zweckmäßig ist, wenn diese Verschlüsse 10 und 11 faltbar sind, damit sie einerseits in den Katheter 7 passen und andererseits durch die Verbindung 2 hindurch eingeführt und an den jeweiligen Anwendungsort gebracht werden können. Mit einer im Bereich des Eintritts in die Innenlängshöhlung 12 angeordneten Öse 16 und einem diese durchsetzenden Zugfaden 17 kann der Sitz des Verschlußelements vor allem durch abwechselnde Betätigung des Fadens 17 und des Mandrins oder Werkzeugs korrigiert werden.

## Patentansprüche

1. Vorrichtung (1) zum Verschließen einer nach der Geburt offen gebliebenen Verbindung (2) zwischen der Aorta (3) und der Arteria pulmonalis (4) mit einem in diese Verbindung mittels Katheter (7) einführbaren Verschlußelement, wobei das Verschlußelement (8) zwei beabstandete, über ein Zugelement (9) elastisch verbundene Verschlüsse (10, 11) aufweist, die in Gebrauchsstellung die beiden Mündungen der zwischen der Aorta (3) und der Arteria pulmonalis (4) befindlichen Verbindung (2) abdecken oder ausfüllen, **dadurch gekennzeichnet, daß** ein Verschluß (11) und das Zugelement (9) eine durchgehende, bis in die Nähe des anderen Verschlusses (10) reichende Innenlängshöhlung (12) für den Eintritt eines Mandrins oder dergleichen Schiebe- und/oder Drehwerkzeuges hat, die an ihrem Ende ausgesteift und/oder armiert ist, und daß im Bereich des Eintrittes in die Innenlängshöhlung (12) des Zugelementes (9) eine Armierung angeordnet ist, an welcher eine über den unmittelbar benachbarten Verschluß (11) nach außen überstehende Öse (16) und ein an dieser eingehängter Zugfaden (17) angeordnet sind.

2. Vorrichtung nach Anspruch 1, **dadurch gekennzeichnet, daß** die Armierung im Bereich des Eintrittes in die Innenlängshöhlung (12) des Zugelementes (9) eine Wendel (15) vorzugsweise aus Metalldraht ist.

3. Vorrichtung nach Ansprüche 1 bis 2, **dadurch gekennzeichnet, daß** die Verschlüsse (10, 11) und das oder die sie verbindenden Zugelemente (9) aus elastischem Kunststoff, insbesondere aus Silikon-Material, bestehen.

4. Vorrichtung nach einem der Ansprüche 1 bis 3, **dadurch gekennzeichnet, daß** die beiden Verschlüsse (10, 11) und das sie elastisch verbindende Zugelement (9) einstücktig verbunden sind.

5. Vorrichtung nach einem der Ansprüche 1 bis 4, **dadurch gekennzeichnet, daß** die beiden Verschlüsse (10, 11) des Verschlußelementes platten- oder schirmartig ausgebildet und eben oder wenigstens bereichsweise an ihrer äußeren Oberfläche konkav eingesenkt sind.

6. Vorrichtung nach einem der Ansprüche 1 bis 5, **dadurch gekennzeichnet, daß** die Innenlängshöhlung (12) des Zugelementes (9) innenseitig armiert ist und daß die Armierung an ihrem Ende eine innenseitige hohle Profilierung oder einen Schlitz bildet oder umgrenzt, in den das flache Ende des Mandrins oder Werkzeuges paßt.

7. Vorrichtung nach einem der Ansprüche 6, **dadurch gekennzeichnet, daß** die Innenlängshöhlung (12) des Zugeleementes (9) durch eine Wendel (13), zum Beispiel aus einem Metalldraht, innenseitig armiert ist.

8. Vorrichtung nach einem der Ansprüche 1 bis 7, **dadurch gekennzeichnet, daß** die schirmartigen Verschlüsse (10, 11) gegen die Elastizität ihres Materials oder Werkstoffes faltbar und in einen schlauchartigen Zuführkatheter (7) einsetzbar sind, dessen Innenquerschnitt der Außenabmessung der zusammengefalteten Verschlüsse (10, 11) entspricht.

9. Vorrichtung nach einem der Ansprüche 1 bis 8, **dadurch gekennzeichnet, daß** die schirmartigen Verschlüsse (10, 11) eine runde, viereckige, vieleckige oder ovale Umfangskontur haben und ihre Fläche insbesondere Aussteifungsrippen oder -stege (18) aufweist.

10. Vorrichtung nach einem der vorstehenden Ansprüche, **dadurch gekennzeichnet, daß** das Zugelement (9) zwischen den beiden endseitigen Verschlüssen (10, 11) wenigstens einen weiteren insbesondere elastischen Verschluß (19) trägt, der zumindest in seinem Randbereich elastisch anpaßbar ist.

11. Vorrichtung nach einem der Ansprüche 1 bis 10, **dadurch gekennzeichnet, daß** an dem Zugelement (9) zwischen den beiden endseitigen Verschlüssen (10, 11) zwei zueinander beabstandete weitere Verschlüsse (19) angeordnet sind, deren Außendurchmesser insbesondere kleiner als der der endseitigen Verschlüsse (10, 11) ist.

12. Vorrichtung nach einem der Ansprüche 1 bis 11, **dadurch gekennzeichnet, daß** der oder die zusätzlichen Verschlüsse (19) im Verlauf des Zugelementes (9) ebenfalls etwa schirmartig und gegen eine Rückstellkraft des Werkstoffes faltbare, schirmartige oder konische Form haben.

## Claims

1. Device (1) for closing a congenitally open connection (2) between the aorta (3) and the pulmonary artery (4), having a closure element which can be inserted in this connection by means of a catheter (7), the closure element (8) having two spaced-apart closures (10, 11) reslilently connected via a tensioning element (9), which in the position of use cover or fill the two openings of the connection (2) located between the aorta (3) and the pulmonary artery (4), **characterised in that** a closure (11) and the tensioning element (9) have a continuous inner longitudinal cavity (12), extending nearly to the other closure (10), for the insertion of a mandrel or similar pushing and/or rotating tool, said cavity being strengthened and/or reinforced at its end, and **in that** in the region of the entry into the inner longitudinal cavity (12) of the tensioning element (9) a reinforcement is provided on which are disposed an eyelet (16) protruding outwards over the immediately adjacent closure (11) and a tensioning thread (17) suspended from said eyelet (16).

2. Device according to claim 1, **characterised in that** the reinforcement in the region of entry into the inner longitudinal cavity (12) of the tensioning element (9) is a coil (15), preferably of metal wire.

3. Device according to claims 1 and 2, **characterised in that** the closures (10, 11) and the tensioning element(s) (9) connecting them consist of a resilient plastic, particularly silicon material.

4. Device according to one of claims 1 to 3, **characterised in that** the two closures (10, 11) and the tensioning element (9) connecting them are integrally joined together.

5. Device according to one of claims 1 to 4, **characterised in that** the two closures (10, 11) of the closure element are plate-shaped or umbrella-shaped and are flat or have a concave depression over at least part of their outer surface.

6. Device according to one of claims 1 to 5, **characterised in that** the inner longitudinal cavity (12) of the tensioning element (9) is reinforced on the inside and **in that** at its end the reinforcement forms or defines an internal hollow profile or a slot into which the flat end of the mandrel or tool fits.

7. Device according to claim 6, **characterised in that** the inner longitudinal cavity (12) of the tensioning element (9) is reinforced on the inside by a coil (13), for example a coil of metal wire.

8. Device according to one of claims 1 to 7, **characterised in that** the umbrella-shaped closures (10, 11) can be folded counter to the elasticity of their material and can be inserted in a tubular insertion catheter (7), the internal cross-section of which corresponds to the outer dimensions of the collapsed closures (10, 11).

9. Device according to one of claims 1 to 8, **characterised in that** the umbrella-shaped closures (10, 11) have a round, rectangular, polygonal or oval circumferential contour and their surface has, in particular, reinforcing ribs or struts (18).

10. Device according to one of the preceding claims, **characterised in that** the tensioning element (9) carries, between the two end closures (10, 11), at least one additional, more particularly elastic, closure (19) which is flexibly adaptable, at least in its edge region.

11. Device according to one of claims 1 to 10, **characterised in that** two additional spaced-apart closures (19) are disposed on the tensioning element (9) between the two end closures (10, 11), the external diameter of said additional closures (9) being, in particular, less than that of the end closures (10, 11).

12. Device according to one of claims 1 to 11, **characterised in that** the additional closure(s) (19) along the tensioning element (9) also have an umbrella-shaped or conical form which can be folded substantially in the manner of an umbrella counter to the resilience of the material.

## Revendications

1. Dispositif (1) pour fermer une communication (2) entre l'aorte (3) et l'artère pulmonaire (4) restée ouverte après la naissance à l'aide d'un élément d'obturation pouvant être introduit dans ce passage au moyen d'un cathéter (7), l'élément d'obturation (8) présentant deux obturateurs (10, 11) distants l'un de l'autre et reliés de manière élastique par un élément de traction (9), qui, en position d'utilisation, recouvrent ou comblent les deux ouvertures de la communication (2) entre l'aorte (3) et l'artère pulmonaire (4), **caractérisé en ce qu'**un obturateur (11) et l'élément de traction (9) possèdent une cavité longitudinale (12) traversante s'étendant jusqu'à proximité de l'autre obturateur (10) pour permettre l'introduction d'un mandrin ou d'un outil coulissant et/ou rotatif analogue, laquelle est renforcée et/ou armée à son extrémité, et **en ce que** dans la zone de l'entrée de la cavité longitudinale (12) de l'élément de traction (9) sont disposés une armature qui porte un oeillet (16) dépassant de l'obturateur (11) directement adjacent vers l'extérieur, et un fil de traction (17) accroché à l'oeillet.

2. Dispositif selon la revendication 1, **caractérisé en ce que** l'armature dans la zone de l'entrée dans la cavité longitudinale (12) de l'élément de traction (9) est une spirale (15) de préférence en fil métallique.

3. Dispositif selon les revendications 1 et 2, **caractérisé en ce que** les obturateurs (10, 11) et l'élément ou les éléments de traction (9) qui les relie(nt) entre eux sont en une matière plastique élastique, notamment en silicone.

4. Dispositif selon l'une des revendications 1 à 3, **caractérisé en ce que** les deux obturateurs (10, 11) et l'élément de traction (9) qui les relie entre eux de façon élastique sont d'un seul tenant.

5. Dispositif selon l'une des revendications 1 à 4, **caractérisé en ce que** les deux obturateurs (10, 11) de l'élément d'obturation sont réalisés en forme de plaquette ou de parapluie et sont plans ou, au moins dans certaines zones de leur surface extérieure, concaves.

6. Dispositif selon l'une des revendications 1 à 5, **caractérisé en ce que** la cavité longitudinale (12) de l'élément de traction (9) est armée à l'intérieur et **en ce que** l'armature forme ou délimite à son extrémité un profil intérieur creux ou une fente dans lequel (laquelle) s'adapte l'extrémité plate du mandrin ou de l'outil.

7. Dispositif selon la revendication 6, **caractérisé en ce que** la cavité longitudinale (12) de l'élément de traction (9) est armée à l'intérieur par une spirale (13), par exemple un fil métallique.

8. Dispositif selon l'une des revendications 1 à 7, **caractérisé en ce que** les obturateurs (10, 11) en forme de parapluie peuvent être pliés en vainquant l'élasticité de la matière ou du matériau qui les constitue et introduits dans un cathéter d'amenée (7) semblable à un tuyau souple dont la section transversale intérieure correspond à la dimension extérieure des obturateurs (10, 11) pliés.

9. Dispositif selon l'une des revendications 1 à 8, **caractérisé en ce que** les obturateurs (10, 11) semblables à des parapluies possèdent un pourtour circulaire, rectangulaire, polygonal ou ovale et **en ce que** leur surface présente notamment des nervures ou des ailes de renforcement (18).

10. Dispositif selon l'une des revendications précédentes, **caractérisé en ce que** l'élément de traction (9) porte entre les deux obturateurs (10, 11) situés aux extrémités un obturateur (19) supplémentaire notamment élastique, qui est adaptable élastiquement au moins dans sa partie périphérique.

11. Dispositif selon l'une des revendications 1 à 10, **caractérisé en ce que** deux obturateurs (19) supplémentaires distants l'un de l'autre sont disposés sur l'élément de traction (9) entre les deux obturateurs (10, 11) situés aux extrémités, dont le diamètre extérieur est notamment inférieur à celui des obturateurs (10, 11) situés aux extrémités.

12. Dispositif selon l'une des revendications 1 à 11, **caractérisé en ce que** l'obturateur ou les obturateurs supplémentaire(s) (19) placés le long de l'élément de traction (9) ressemblent également approximativement à un parapluie et ont une forme de cône ou de parapluie pouvant être plié contre une force de rappel du matériau.
